# DEMANDE DE BREVET EUROPEEN

(11) **EP 1 655 057 A1**
(43) Date de publication de la demande: **10.05.2006**
(21) Numéro de dépôt: 05292284.6
(22) Date de dépôt: 27.10.2005
(51) Int. Cl.: A61Q 19/06, A61K 8/34, A61K 8/55, A61K 8/41

(54) **Solutions aqueuses pour la réduction de tissus graisseux**

(30) Priorité: 27.10.2004 FR 0411474
(71) Demandeur: Bernstein, Serge, 93800 Epinay sur Seine (FR); Crassas, Yves, 69006 Lyon (FR)
(72) Inventeur: Bernstein, Serge, 93800 Epinay sur Seine (FR); Crassas, Yves, 69006 Lyon (FR)
(74) Mandataire: Colombet, Alain André

(57) **Abrégé**

La présente invention concerne des solutions aqueuses isotoniques ou hypotoniques, hyperkaliémiques ou contenant du tiratricol et/ou de la phosphatidylcholine, et leur utilisation pour la réduction de tissus graisseux.

## Description

La présente invention concerne des solutions aqueuses utilisables pour la réduction de tissus graisseux, en particulier une solution isotonique ou hypotonique, hyperkaliémique ou contenant du tiratricol et/ou un composé amphotère comportant un pôle lipophile et un pôle hydrophile tel que la phosphatidylcholine ainsi que leur utilisation pour la réduction de tissus graisseux, en particulier dans une méthode de traitement esthétique de la cellulite par lipotomie.

Dans le cadre de la présente invention, le terme "cellulite" désigne, conformément au langage courant, une cellulite esthétique, c'est à dire une hypertrophie du tissu adipeux généralement très localisée formant des bosselures appelées "peau d'orange".

La lipotomie, ou traitement hypo-osmolaire, est une technique permettant de réduire la cellulite qui implique une hyperhydratation intra-cellulaire, une fragilisation membranaire ou une tonicité potassique des adipocytes pour permettre leur destruction. Cette technique repose sur un principe de dilution du liquide interstitiel baignant les adipocytes, de manière à induire des différences de pression osmotique de part et d'autre de la membrane des adipocytes. Ces différences de pression osmotique se traduisent par un flux de liquide depuis le compartiment de plus faible osmolarité (le liquide interstitiel) vers le compartiment de plus forte osmolarité (le cytoplasme des adipocytes). La technique a été initialement développée en France par le Dr Serge Berstein (demande de brevet français FR 97 14073) et aux Etats-Unis par le Docteur Steven Hoefflin (Aesthetic Surgery, 2002 22(6)).

L'osmole (Osm) est une unité physique de mesure de la pression osmotique exprimée en mole par litre. Lorsqu'une mole d'une substance est dissoute dans un litre d'eau, elle exerce une pression osmotique d'une osmole. L'osmolarité est la concentration en substance dissoute exerçant un pouvoir osmotique. L'osmolarité du plasma est considérée comme étant comprise entre 280 mOsm/l et 330 mOsm/l. L'osmolarité du cytoplasme cellulaire est de 394 mOsm/1.

Les termes "hypotonie", "isotonie" et "hypertonie" sont utilisés ici pour désigner un compartiment dont l'osmolarité est inférieure, égale et supérieure, respectivement, à l'osmolarité du plasma.

La technique de lipotomie classiquement utilisée comprend l'administration intra-graisseuse d'une solution dite "de destruction", fortement hypotonique (d'une osmolarité généralement comprise entre 120 mOsm/l et 20 mOsm/l), qui provoque une hyperhydratation des adipocytes. L'augmentation de volume des adipocytes entraîne une fragilisation de leur membrane, voire des lésions cellulaires. La destruction des adipocytes est complétée par un traumatisme mécanique léger, par exemple par application d'un champ ultrasonore transcutané ou par massage de type palper-rouler notamment. Les ultrasons génèrent une dépression provoquant l'"explosion" des cellules par cavitation. La lésion puis la destruction des adipocytes conduit à un relargage des graisses ainsi que des triglycérides, acides gras libres et autres contenus cellulaires dans le plasma avant d'être dégradés par le foie pour être éliminés. Les débris cellulaires sont évacués par le système lymphatique.

Cette étape de destruction ne permet toutefois que de traiter un volume limité de tissu graisseux. Au-delà de un à deux centimètres d'épaisseur de tissu graisseux, l'ensemble des adipocytes ne peut être ciblé. Or la réitération de l'étape de destruction est problématique. En effet l'emploi d'une solution de très basse osmolarité induit l'installation d'une fibrose tissulaire qui nécessite environ 2 à 3 mois pour se résorber. Une nouvelle administration d'une solution fortement hypotonique ne pouvant être mise en oeuvre avant résorption de la fibrose, cette étape de destruction, si elle devait être répétée, conduirait à un traitement esthétique par lipotomie d'une durée beaucoup trop longue. En outre, une récidive est souvent observée 2 à 3 mois après l'étape de destruction. Des traitements aussi espacés nuiraient donc à l'efficacité globale de la méthode.

La récidive est par ailleurs prévenue à l'aide d'un traitement dit "de suite" qui comprend jusqu'à trois administrations, à environ un mois d'intervalle, d'une solution peu hypotonique (environ 200 mOsm/l) qui permet de libérer les protéines emprisonnées dans le tissu fibreux et exerce ainsi un effet drainant.

Dans le cas de personnes présentant un volume de graisse trop important pour que la méthode de traitement esthétique par lipotomie soit pleinement efficace, on associe une liposuccion qui permet à la fois d'éliminer les graisses émulsionnées devant être absorbées ainsi que des adipocytes qui n'auraient pas été détruits. La liposuccion est toutefois une technique invasive, plus traumatisante que la lipotomie, et il est donc préférable de pouvoir s'en passer.

La présente invention vise donc à améliorer les méthodes de traitement esthétique par lipotomie actuellement utilisées de manière à éviter de répéter l'administration de solutions fortement hypotoniques et/ou de devoir recourir à une liposuccion complémentaire, lorsque le volume de graisse à éliminer est trop important. Un volume de graisse est considéré comme trop important lorsque l'épaisseur du tissu graisseux est déterminée par échographie comme étant supérieure à un ou deux centimètres. Il va de soi que les améliorations apportées par la présente invention sont également utiles lorsque le volume de graisse est moindre, puisqu'elles contribuent à augmenter l'efficacité de la lipotomie.

Les inventeurs ont ainsi mis au point une solution iso- ou hypotonique et hyperkaliémie dont l'administration au cours d'une méthode de traitement esthétique par lipotomie, en tant que préalable à la séance de destruction, permet de diminuer le volume des adipocytes et ainsi de cibler un maximum de cellules à la fois au cours de l'étape de destruction. L'administration de cette solution pouvant être répétée à brève échéance, elle permet de réduire la durée totale de la méthode de lipotomie. En outre, grâce à l'emploi de cette solution, le volume de graisse traité lors de la séance de destruction est moindre, et le recours à la liposuccion n'est donc plus nécessaire.

Les inventeurs ont par ailleurs mis en évidence qu'une synergie d'action peut également être observée entre une solution isotonique hyperkaliémique, ou une solution hypotonique éventuellement hyperkaliméique, ou et certaines substances telles que le tiratricol (acide triiodothyroacétique) ou un composé amphotère tel que la phosphatidylcholine.

### Solution iso- ou hypotonique hyperkaliémique

Les inventeurs ont développé une solution isotonique ou hypotonique et hyperkaliémique qui permet, lorsque nécessaire, de réduire massivement le tissu graisseux jusqu'à une épaisseur de 1 à 2 cm, épaisseur à partir de laquelle un traitement par une solution de destruction peut être mis en oeuvre avec suffisamment d'efficacité pour ne pas devoir être répété.

Le potassium est un cation qui présente une répartition essentiellement intracellulaire (98% contre 2% dans le milieu extracellulaire). La kaliémie, c'est-à-dire la concentration de potassium dans le plasma, est d'environ 4 mmol/l, ce qui correspond à une osmolarité liée au potassium de 4 mOsm/l. L'osmolarité intracellulaire liée au potassium dans les adipocytes est d'environ 160 mOsm/l. Le maintien de cette répartition inégale de part et d'autre des membranes plasmiques est assurée grâce à un transport actif via des pompes Na, K et ATP. Dans le contexte de la demande, on désigne par "solution hyperkaliémique" une solution dans laquelle la concentration en potassium est supérieure à celle du plasma ; de préférence, il s'agit d'une solution comprenant 20 à 110 mOsm/l, de préférence encore comprenant 25 à 110 mOsm/l de potassium.

Du fait de ce transport actif, le potassium ajouté à une solution hypotonique serait transporté et accumulé dans les adipocytes, contribuant ainsi à accentuer le gradient de pression osmotique entre le milieu interstitiel, dilué par la solution hypotonique, et le milieu intracellulaire. Le potassium est par ailleurs cytotoxique pour les cellules lorsque sa concentration est importante. Un rapport K/Na supérieur à 1 augmente l'efficacité.

Toutefois, l'utilisation d'une concentration de potassium suffisamment élevée pour observer un effet accru de réduction du tissu graisseux dans une solution hypotonique, efficace en lipotomie, n'est pas possible. En effet, l'osmolarité des solutions utilisées est tellement basse (les solutions de destruction présentent une osmolarité de 60-90 mOsm/l) que l'accentuation de l'effet de destruction par le potassium pourrait conduire à des d'accidents, avec le risque d'une nécrose graisseuse et cutanée nécessitant une exérèse chirurgicale. Or les inventeurs ont constaté que, par rapport à une solution de destruction d'une osmolarité de 60 mOsm/l, l'association de potassium avec une solution de destruction d'osmolarité plus élevée n'offre pas d'avantage en terme de récupération tissulaire et ne diminue pas pour autant le risque de nécrose et de fibrose.

Les inventeurs ont mis en évidence qu'un gain réel en terme de récupération tissulaire permettant des traitements itératifs sans risque n'est observé qu'à partir d'une osmolarité de 167 mOsm/l, pour des solutions hyperkaliémiques. Les mêmes solutions hypotoniques dépourvues de potassium ne présentent pas d'efficacité en terme de réduction des graisses.

Les inventeurs ont développé une solution modérément hypotonique ou isotonique (d'osmolarité comprise entre 167 et 330 mOsm/l) contenant du potassium à hauteur environ de 20 à 110 mOsm/l, éventuellement associé à un autre agent dont la pénétration intracellulaire est facilitée par l'augmentation de la porosité membranaire due à l'hyperhydratation et à l'augmentation du volume cellulaire. Cette administration constitue une étape préparatoire de la lipotomie, lorsque le volume de graisse à éliminer est trop important, c'est-à-dire supérieur à 1-2 cm d'épaisseur comme déterminé par échographie.

L'invention propose plus particulièrement une solution aqueuse hypotonique ayant une osmolarité comprise entre 167 mOsm/l et 257 mOsm/l et comprenant de 20 à 110 mOsm/l de potassium. Dans le cadre de cette demande, on appelle cette solution "solution préparative".

Préférentiellement, l'osmolarité de la solution est comprise entre 227 et 257 mOsm/l.

Préférentiellement, l'osmolarité du potassium dans la solution est comprise entre 25 et 110 mOsm/l, de préférence entre 28 et 107 mOsm/l, de préférence entre 40 et 70 mOsm/l, de préférence encore entre 50 et 60 mOsm/l. Selon un mode de réalisation préféré, la solution contient 53,6 mOsm/l de potassium.

Les solutions utilisées en lipotomie étant injectées directement dans le tissu graisseux, pour éviter qu'elles ne diffusent et donc que l'effet de dilution soit amoindri, on administre préalablement ou conjointement un composé vasoconstricteur, généralement de l'adrénaline, pour isoler vasculairement le tissu gras. Avantageusement, le vasoconstricteur peut être directement dilué dans la solution préparative selon l'invention. Le choix d'une concentration appropriée est à la portée de l'homme du métier. Par exemple, la solution préparative selon l'invention peut comprendre en outre 1,2 mOsm/l d'adrénaline.

De la même manière, un agent anesthésiant peut être administré préalablement ou conjointement à la solution préparative. Des exemples d'anesthésiants utilisés en lipotomie incluent la lidocaïne ou la procaïne. Préférentiellement, l'anesthésiant est directement dilué dans la solution préparative, par exemple avec une osmolarité de 13,2 mOsm/l.

D'autre part, la solution préparative selon l'invention peut comprendre du bicarbonate, qui est classiquement utilisé pour contrebalancer l'acidose qui peut résulter de la lésion ou de la destruction des adipocytes, et/ou du chlorure de sodium. L'adjonction de calcium dans le soluté potentialise les effets destructeurs.

Ainsi selon un mode de réalisation préféré, la solution préparative selon l'invention a une osmolarité comprise entre 167 mOsm/l et 257 mOsm/l, de préférence entre 227 et 257 mOsm/l, et comprend de 50 à 60 mOsm/l de potassium, et éventuellement 13,2 mOsm/l de xylocaïne et/ou 1,2 mOsm/l d'adrénaline.

Par exemple on peut obtenir une solution d'osmolarité égale à 257 mOsm/l et comprenant 53,6 mOsm/l de potassium, en ajoutant 10 ml de xylocaïne à 2% (soit 3,3 mOsm), 1ml d'adrénaline à 1 mg (soit 0,3 mOsm), 10 ml de KCl à 10% (soit 26,8 mOsm), et 17 ml de bicarbonate de sodium à 8,4% (soit 34 mOsm) dans de l'eau pour préparation injectable en quantité suffisante pour 250 ml.

La composition peut en outre comprendre des composés tels que la caféine et/ou un ou plusieurs corticoïdes.

Comme la solution préparative est modérément hypotonique ou isotonique, elle ne provoque pas de dommages tissulaires, en particulier de nécrose, et l'oedème causé par son administration régresse rapidement. Cette solution peut donc être administrée à intervalles de temps courts. Si l'emploi de cette solution permet de réduire efficacement l'épaisseur du tissu graisseux, on observe toutefois une récidive précoce, dès 3 semaines après traitement, si bien que cette solution ne peut pas représenter en soi la seule base de traitement. L'utilisation de la solution selon l'invention représente donc une étape de préparation du tissu graisseux avant l'étape de destruction, réalisée en général par combinaison de l'administration d'une solution fortement hypo-osmolaire et par application d'un traumatisme léger (ultrasons, massage).

L'invention concerne également l'utilisation d'une solution aqueuse ayant une osmolarité comprise entre 167 mOsm/l et 330 mOsm/l et comprenant de 25 à 110 mOsm/l de potassium, telle que décrite ci-dessus, pour la réduction de tissu graisseux, en particulier pour le traitement esthétique de la cellulite.

### Solution iso- ou hypotonique contenant du tiratricol et/ou de la phosphatidylcholine

Les inventeurs ont par ailleurs mis en évidence qu'une synergie d'action peut également être observée entre une solution isotonique hyperkaliémique, ou une solution hypotonique éventuellement hyperkaliémique, et certaines substances telles que le tiratricol (acide trüodothyroacétique) ou un composé amphotère tel que la phosphatidylcholine.

Les inventeurs ont mis en évidence qu'un effet de réduction des graisses avec un temps de récupération tissulaire amélioré pouvait également être obtenu avec une solution hyperkaliémique isotonique ou modérément hypotonique (ayant une osmolarité comprise environ entre 120 et 330 mOsm/l) contenant du tiratricol et/ou de la phosphatidylcholine. Une telle solution est donc également appropriée pour une préparation du tissu graisseux avant destruction par lipotomie.

Les inventeurs ont aussi montré que la phosphatidylcholine et/ou le tiratricol, plus spécifiquement la phosphatidylcholine, permettaient d'augmenter l'efficacité d'une solution de lipotomie, fortement hypotonique (environ 20 à 90 mOsm/l), sur la destruction du tissu graisseux.

L'invention concerne donc une solution aqueuse hypotonique ou isotonique hyperkaliémique, ayant une osmolarité comprise entre 20 mOsm/l et 330 mOsm/l et comprenant entre 1000 mg/l et 4800 mg/l de phosphatidylcholine et/ou entre 13,2 mg/l et 26,4 mg/l de tiratricol. Plus spécifiquement, il peut s'agir d'une solution aqueuse ayant une osmolarité comprise entre 167 mOsm/l et 330 mOsm/l et comprenant de 20 à 110 mOsm/l de potassium, telle que décrite ci-dessus, et comprenant en outre entre 1000 mg/l et 4800 mg/l de phosphatidylcholine et/ou entre 13,2 mg/l et 26,4 mg/l de tiratricol.

Selon un mode de réalisation la solution aqueuse selon l'invention a une osmolarité comprise entre 20 et 90 mOsm/l. Selon un autre mode de réalisation la solution aqueuse selon l'invention a une osmolarité comprise entre 120 et 330 mOsm/l.

De préférence la solution aqueuse selon l'invention comprend entre 1000 mg/l et 1200 mg/l de phosphatidylcholine. De préférence la solution aqueuse selon l'invention comprend 13,2 mg/l de tiratricol. De préférence encore la solution aqueuse selon l'invention comprend 1000-1200 mg/l de phosphatidylcholine et 13,2 mg/l de tiratricol.

Pour une utilisation pour la réduction de l'épaisseur du tissu graisseux avant destruction par lipotomie, on préfère une solution ayant une osmolarité comprise entre 120 et 330 mOsm/l, comprenant entre 13,2 mg/l et 26,4 mg/l de tiratricol, de préférence 13,2 mg/l, et/ou entre 1000 mg/l et 4800 mg/l de phosphatidylcholine, de préférence entre 1000 mg/l et 1200 mg/l.

Pour une utilisation en tant que solution de destruction par lipotomie, on préfère une solution ayant une osmolarité comprise entre 20 et 90 mOsm/l, comprenant entre 1000 mg/l et 4800 mg/l de phosphatidylcholine, de préférence entre 1000 mg/l et 1200 mg/l, et/ou entre 13,2 mg/l et 26, 4 mg/l de tiratricol, de préférence 13,2 mg/l.

L'invention concerne également l'utilisation de ces solutions dans un traitement de la cellulite par lipotomie.

### Méthode de traitement esthétique par lipotomie

L'invention propose une méthode de traitement esthétique de la cellulite, par lipotomie, améliorée par rapport aux méthodes existantes. Cette méthode permet de supprimer et/ou réduire efficacement la cellulite, en particulier lorsque l'épaisseur initiale du tissu graisseux est supérieure à 1 à 2 cm, comme déterminé par échographie (méthode de référence). L'épaisseur du tissu graisseux peut également être déterminée par toute autre méthode appropriée telle qu'une radiographie des tissus mous ou un scanner.

La méthode de traitement esthétique de la cellulite selon l'invention comprend les étapes consistant à :
a) administrer une solution aqueuse ayant une osmolarité comprise entre 167 mOsm/l et 330 mOsm/l et comprenant 20 à 110 mOsm/l de potassium, telle que définie précédemment dans un tissu graisseux ;
b) éventuellement répéter l'étape a) jusqu'à ce que l'épaisseur du tissu graisseux, déterminée par échographie, soit inférieure ou égale à 2 cm, notamment comprise entre 1 et 2 cm ;
c) administrer une solution aqueuse ayant une osmolarité comprise entre 20 mOsm/l et 90 mOsm/l dans ledit tissu graisseux ;
d) appliquer audit tissu un traitement mécanique susceptible de provoquer la destruction de la membrane d'adipocytes présents dans ledit tissu graisseux ; et
e) éventuellement, administrer une solution ayant une osmolarité comprise entre 180 et 200 mOsm/l dans ledit tissu graisseux traité par le traitement mécanique.

De manière préférentielle, la solution hyperkaliémique utilisée à l'étape a) est hypotonique, avec une osmolarité comprise entre 167 mOsm/l et 257 mOsm/l, de préférence entre 227 mOsm/l et 257 mOsm/l. L'osmolarité du potassium dans cette solution est de préférence comprise entre 25 et 110 mOsm/l, de préférence entre 40 et 70 mOsm/l, de préférence encore entre 50 et 60 mOsm/l, ou encore égale à 53,6 mOsm/l.

La solution utilisée à l'étape a) peut comprendre en outre un vasoconstricteur, un anesthésiant, du bicarbonate et/ou du chlorure de sodium, comme décrit dans la présente demande au sujet de la solution préparative.

Selon un mode de réalisation préféré, la solution administrée à l'étape a) a une osmolarité comprise entre 167 mOsm/l et 257 mOsm/l, de préférence entre 227 et 257 mOsm/l, et comprend 50 à 60 mOsm/l de potassium, et éventuellement 13,2 mOsm/l de xylocaïne et/ou 1,2 mOsm/l d'adrénaline.

De manière alternative, on peut utiliser, à la place de la solution isotonique ou hypotonique hyperkaliémique de l'étape a), une solution aqueuse isotonique hyperkaliémique ou une solution hypotonique, ayant une osmolarité comprise entre 120 et 330 mOsm/l, et comprenant entre 13,2 mg/l et 26,4 mg/l de tiratricol, de préférence 13,2 mg/l, et éventuellement entre 1000 mg/l et 4800 mg/l de phosphatidylcholine, de préférence entre 1000 mg/l et 1200 mg/l.

L'étape a) peut être répétée autant que nécessaire, à intervalle de une à deux semaines, de préférence toutes les semaines, jusqu'à ce que le tissu graisseux traité présente une épaisseur comprise entre 1 et 2 cm, comme déterminé par échographie. Cette échographie est réalisée selon les critères standard, en mesurant l'épaisseur de l'hypoderme compris entre le derme et l'aponévrose musculaire.

Cette étape conduit à une réduction du volume des adipocytes ce qui permet, lors de l'application ultérieure du traitement selon l'étape c) et d), de traiter un maximum de cellules dans le volume de tissu graisseux accessible à ces traitements.

L'étape c) peut être mise en oeuvre environ une à deux semaines, de préférence une semaine, après la dernière administration de la solution "préparative" d'osmolarité comprise entre 167 mOsm/l et 330 mOsm/l et comprenant 20 à 110 mOsm/l de potassium.

Avantageusement, la solution utilisée à l'étape c) (solution de destruction) peut comprendre un vasoconstricteur, un anesthésiant, du bicarbonate et/ou du chlorure de sodium. Préférentiellement cette solution comprend 13,2 mOsm/l de xylocaïne et/ou 1,2 mOsm/l d'adrénaline. Cette solution peut aussi comprendre entre 1000 mg/l et 4800 mg/l de phosphatidylcholine, de préférence entre 1000 mg/l et 1200 mg/l, et éventuellement entre 13,2 mg/l et 26,4 mg/l de tiratricol, de préférence 13,2 mg/l.

Un exemple de solution de destruction utilisable pour mettre en oeuvre l'étape c) comprend 10 ml de xylocaïne 2%, 1 ml d'adrénaline à 1 mg et 2,5 ml de NaCl 10% ou 4,2 ml de bicarbonate de sodium 8,4%, dans de l'eau pour préparation injectable en quantité suffisante pour 250 ml.

Parallèlement à une diminution de volume, le traitement préalable selon l'étape a) provoque dans certains cas l'apparition d'une zone facilement compressible et lâche au toucher. Avantageusement, l'osmolarité totale de la solution de destruction utilisée à l'étape c) peut être ajustée en fonction du "coefficient de compressibilité" du tissu. Ce coefficient de compressibilité est défini comme le rapport de l'épaisseur du tissu sous-cutané mesurée par échographie, sans exercer de pression sur le tissu, sur l'épaisseur minimale du même tissu, comme mesurée par l'opérateur en comprimant au maximum le tissu avec la sonde d'échographie. Par exemple, lorsque le coefficient de compressibilité est compris entre 1 et 1,5, on peut utiliser une solution dont l'osmolarité totale est comprise entre 90 et 75 mOsm/l; pour un coefficient de compressibilité compris entre 1,5 et 2,5, on peut avantageusement utiliser une solution d'osmolarité comprise entre 75 et 60 mOsm/l ; pour un coefficient de compressibilité supérieur 2,5, on peut avantageusement utiliser une solution d'osmolarité comprise entre 60 et 20 mOsm/l.

Un traitement mécanique susceptible de provoquer la destruction de la membrane d'adipocytes présents dans le tissu graisseux peut consister à appliquer un champ d'ultrasons transcutané audit tissu graisseux, dans des conditions suffisantes pour obtenir une destruction d'adipocytes et/ou à masser le tissu graisseux par des massages de type palper-rouler, par exemple.

L'application d'un champ ultrasonore, sur des adipocytes hyperhydratés, permet de détruire les adipocytes par cavitation. En général, le traitement par les ultrasons peut être mis en oeuvre immédiatement après administration de la solution hypotonique de destruction. On peut s'assurer d'un état d'hydratation suffisant des adipocytes en contrôlant que le tissu graisseux présente une fermeté accrue par rapport à la fermeté constatée avant administration de la solution hypotonique de l'étape c). La détermination des modalités d'utilisation des ultrasons est à la portée de l'homme du métier. Généralement, on utilise un appareil ultrasons basses fréquences en limitant la puissance émise par le transducteur à 3 Watt moyens/cm². Du fait de cette limitation de la puissance, deux transducteurs de fréquence de résonance très proche peuvent être utilisés pour améliorer l'amplitude de l'effet de cavitation destructeur du tissu graisseux, comme décrit dans la demande de brevet FR2809018. L'application se fait généralement pendant 3 à 6 minutes. L'utilisation d'un appareil ultrasonore d'1 méga Hz est possible.

Avantageusement, la méthode selon l'invention comprend en outre l'administration d'une solution dite "de traitement de suite" ayant une osmolarité comprise entre 180 et 200 mOsm/1, de préférence environ égale à 200 mOsm/l. Cette solution peut être administrée après résorption de la fibrose induite par le traitement des étapes c) et d), c'est-à-dire environ 1 mois mise en oeuvre des étapes c) et d). L'administration de cette solution de traitement de suite peut être répétée. Généralement, Le traitement de suite comprend trois administrations, à un environ un mois d'intervalle, d'une solution de traitement de suite.

La détermination du volume de solution à administrer selon les étapes a), b) et c) est à la portée de l'homme du métier. Ces solutions sont administrées par injection transcutanée. A titre d'exemple, on peut procéder à une administration selon un quadrillage de la peau recouvrant le tissu graisseux traité selon des points d'injection répartis à 5 cm d'intervalle sur des lignes parallèles, elles-mêmes distantes de 3 cm. Selon cette modalité, les solutions peuvent être administrées à hauteur de 5 ml de solution par centimètre d'épaisseur du tissu graisseux.

Avantageusement les solutions de préparation, de destruction ou de traitement de suite sont administrées à une température de 38-40°C.

## Revendications

1. Solution aqueuse ayant une osmolarité comprise entre 167 mOsm/l et 330 mOsm/l et comprenant de 20 à 110 mOsm/l de potassium.

2. Solution aqueuse selon la revendication 1, ayant une osmolarité comprise entre 167 mOsm/l et 257 mOsm/l.

3. Solution aqueuse selon la revendication 1 ou 2, ayant une osmolarité comprise entre 227 mOsm/l et 257 mOsm/l.

4. Solution aqueuse selon l'une quelconque des revendications 1 à 3, comprenant de 25 à 110 mOsm/l de potassium.

5. Solution aqueuse l'une quelconque des revendications 1 à 4, comprenant de 40 à 70 mOsm/l de potassium.

6. Solution aqueuse l'une quelconque des revendications 1 à 5, comprenant de 50 à 60 mOsm/l de potassium.

7. Solution aqueuse selon l'une quelconque des revendications 1 à 6, comprenant en outre 13,2 mOsm/l de xylocaïne et/ou 1,2 mOsm/l d'adrénaline.

8. Solution aqueuse selon l'une quelconque des revendications 1 à 7, comprenant en outre 1000 mg/l et 4800 mg/l de phosphatidylcholine et/ou entre 13,2 mg/l et 26,4 mg/l de tiratricol.

9. Solution aqueuse ayant une osmolarité comprise entre 20 mOsm/l et 330 mOsm/l et comprenant entre 1000 mg/l et 4800 mg/l de phosphatidylcholine et/ou entre 13,2 mg/l et 26,4 mg/l de tiratricol.

10. Solution aqueuse selon la revendication 9, ayant une osmolarité comprise entre 120 et 330 mOsm/l.

11. Solution aqueuse selon la revendication 9, ayant une osmolarité comprise entre 20 et 90 mOsm/l.

12. Utilisation d'une solution aqueuse selon l'une quelconque des revendications 1 à 11, pour le traitement esthétique de la cellulite.

13. Méthode de traitement esthétique de la cellulite comprenant les étapes consistant à :
a) administrer une solution aqueuse ayant une osmolarité comprise entre 167 mOsm/l et 330 mOsm/l et comprenant 20 à 110 mOsm/l de potassium, telle que définie dans l'une quelconque des revendications 1 à 11, dans un tissu graisseux ;
b) éventuellement répéter l'étape a) jusqu'à ce que l'épaisseur du tissu graisseux, déterminée par échographie, soit inférieure ou égale à 2 cm, notamment comprise entre 1 et 2 cm ;
c) administrer une solution aqueuse ayant une osmolarité comprise entre 20 mOsm/l et 90 mOsm/l dans ledit tissu graisseux ;
d) appliquer audit tissu un traitement mécanique susceptible de provoquer la destruction de la membrane d'adipocytes présents dans ledit tissu graisseux ; et
e) éventuellement, administrer une solution ayant une osmolarité comprise entre 180 et 200 mOsm/l dans ledit tissu graisseux traité par le traitement mécanique.
